# EUROPEAN PATENT APPLICATION

(11) **EP 4 767 983 A1**
(43) Date of publication of application: **01.07.2026**
(21) Application number: 25227340.4
(22) Date of filing: 29.12.2025
(51) Int. Cl.: A61B 34/20, A61B 5/06, A61B 18/14

(54) **REAL-TIME PROXIMITY FEEDBACK IN INTRALUMINAL CATHETER THERAPY**

(30) Priority: 30.12.2024 IL 31811224; 17.12.2025 US 202519423354
(71) Applicant: Biosense Webster (Israel) Ltd., 2066717 Yokneam (IL)
(72) Inventor: MASSARWA, Fady, 2066717 Yokneam (IL); SEGEV, Meytal, 2066717 Yokneam (IL)
(74) Representative: Carpmaels & Ransford LLP

(57) **Abstract**

A computer-implemented method provides real-time visual feedback of intraluminal catheter engagement within a luminal organ of a patient. A catheter with electrodes on a distal end assembly is positioned in the organ, and a three-dimensional (3D) model of the internal surface is generated and rendered as a 3D anatomical map. For each electrode, a proximity value indicative of distance to a tissue wall is determined based at least on measured impedance and translated into a graphical attribute value. A continuous proximity visualization feature is formed by sequencing the graphical attributes according to electrode positions, representing proximity variations. The feature is projected onto the 3D anatomical map, creating an integrated graphical representation displayed on a computer display. The visualization is dynamically updated in response to impedance changes, providing real-time feedback on catheter contact with the tissue wall.

## Description

### TECHNOLOGICAL FIELD

The presently disclosed subject matter relates to intraluminal catheter therapy.

### BACKGROUND

Intraluminal catheter therapy (ICT) or catheterization has become a pivotal instrument in the field of medicine for both the diagnosis and treatment of various medical disorders. This minimally invasive procedure involves guiding a slender, flexible tube, or catheter, into a luminal organ. Equipped with electrodes, the catheter is used, *inter alia,* for mapping the lumen, and identifying precise locations related to abnormal medical conditions.

Cardiac ICT, a particular example of ICT, is an important tool for both the diagnosis and treatment of cardiac disorders, particularly arrhythmias. This involves inserting a catheter equipped with electrodes through the blood vessels and into the heart, using the catheter for generating a map of the heart's electrical activity, and identifying the precise locations of abnormal electrical activity. The identified sites can then be treated through ablation, where targeted energy neutralizes the abnormal tissue, restoring normal heart rhythm. This integrated approach has revolutionized cardiac care, offering patients less invasive options with shorter recovery times.

### OVERVIEW

A key aspect of catheterization is ensuring precise and effective interaction between the distal end assembly of the catheter and the targeted tissue or anatomical structure. Proximity detection of the catheter's electrodes to the tissue walls of the organ is crucial for accurate and effective results.

A medical practitioner (e.g., physician) manipulating a catheter would benefit from knowing the contact level of the catheter with a tissue wall, in other words, how much force is being applied on the tissue wall. The force distribution on a distal end assembly can help the physician to accurately steer the end. Further, it is desired to avoid the application of excessive force on the tissue when manipulating the catheter, as this may result in deformation of the tissue surface, e.g., tenting, and consequently cause inefficient operation. An indication of force being applied on the tissue walls is also helpful during various other procedures, such as Pulsed Field Ablation (PFA). Too little contact can render the ablation ineffective, while too much pressure can cause excessive tissue damage.

Feedback on catheter engagement with tissue is critical in all these contexts. Engagement includes proximity measurements, which directly indicate the distance to the tissue, and force, which can be inferred from variations in these measurements. This feedback enables the physician to dynamically adjust the catheter's positioning and applied force, supporting precise catheter navigation and effective treatment. By utilizing impedance measurements to dynamically adjust the visual appearance of graphical indications representing electrode proximity, the system enables physicians to make precise, real-time adjustments to the catheter's positioning and applied force. This ensures accurate mapping, effective ablation, and lower risk of tissue damage. For instance, low impedance may prompt repositioning to improve tissue contact, while high impedance may signal excessive pressure, necessitating a reduction in force to prevent tissue tenting or damage.

Existing visualization techniques utilize graphical representations, in addition to that of the catheter itself, often resulting in crowded and visually dense displays. Since the catheter is typically positioned within the volume of the heart, the anatomical map representing the heart's internal structures can sometimes obscure the catheter's displayed data. Rendering the map as semi-transparent is a common approach applied to deal with this problem, but this can complicate depth perception and further hinder usability.

The presently disclosed subject matter includes a computer system, method, and graphical user interface (GUI) that provide real-time, continuous graphical proximity feedback according to changes in electrical impedance sensed by electrodes of a catheter. Unlike conventional approaches, this system shifts visual feedback to the anatomical map by applying a continuous visualization that dynamically represents proximity data directly on the anatomical map in a seamless and uninterrupted manner on the mapped surface.

By presenting real-time and continuous visual feedback on the anatomical map, rather than, for example, around the catheter's representation, the system provides a clearer, more interpretable display that avoids visual clutter and improves the physician's ability to interpret and respond to proximity information.

### BRIEF DESCRIPTION OF THE DRAWINGS

In order to understand the presently disclosed subject matter and to see how it may be carried out in practice, the subject matter will now be described, by way of non-limiting examples only, with reference to the accompanying drawings, in which:
FIG. 1 is a schematic pictorial illustration and example of a catheter-based electrophysiology mapping and ablation system 10;
FIG. 2 is a block diagram schematically illustrating a computer system configured to provide graphical ICT proximity feedback, in accordance with examples of the presently disclosed subject matter;
FIG. 3 is a block diagram schematically illustrating a real-time proximity feedback engine implemented as part of the computer system shown in FIG. 2, in accordance with examples of the presently disclosed subject matter;
FIG. 4 is a flowchart of operations carried out for providing continuous visual proximity feedback, in accordance with examples of the presently disclosed subject matter;
FIG. 5 is a graph demonstrating an impedance-proximity profile, in accordance with examples of the presently disclosed subject matter;
FIG. 6 depicts examples of continuous visual proximity feedback implementations, as disclosed herein;
FIG. 7 is a flowchart of operations carried out for providing directional proximity feedback, in accordance with examples of the presently disclosed subject matter;
FIG. 8 depicts an example of directional proximity feedback, as disclosed herein;
   and
FIG. 9 is a flowchart of operations carried out for a proximity derived dynamic model update procedure, in accordance with examples of the presently disclosed subject matter.

### DETAILED DESCRIPTION

In the following detailed description, numerous specific details are set forth in order to provide a thorough understanding. However, it will be understood by those skilled in the art that the presently disclosed subject matter may be practiced without these specific details. In other instances, well-known methods and features have not been described in detail so as not to obscure the presently disclosed subject matter.

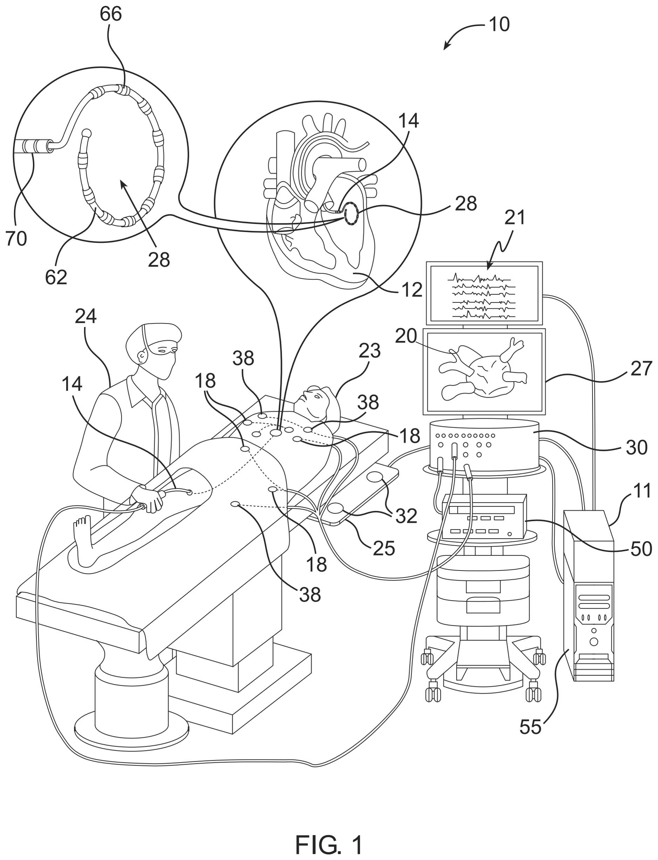

Attention is drawn to FIG. 1 showing an example of an ICT system. More specifically, FIG. 1 shows a catheter-based electrophysiology mapping system 10. In some cases, system 10 can be also used for ablation. System 10 includes one or more catheters, which are percutaneously inserted by a physician 24 through the patient's vascular system into a chamber or vascular structure of a heart 12. Typically, a delivery sheath catheter is inserted into the left or right atrium near a desired location in heart 12. Thereafter, one or more catheters may be inserted into the delivery sheath catheter to arrive at the desired location in heart 12. Catheter types may include, for example, catheters dedicated for sensing Intracardiac Electrogram (IEGM) signals, catheters dedicated for ablating, and/or catheters dedicated for both sensing and ablating. An example catheter 14 is illustrated herein. In some examples physician 24 places a distal end assembly 28 of catheter 14 in contact with the heart wall for sensing a target site in heart 12. For ablation, physician 24 may similarly place a distal end of an ablation catheter in contact with a target site for ablating tissue.

Catheter 14 is an exemplary catheter that includes a distal assembly, having one and preferably multiple electrodes 66 optionally distributed over a plurality of frame elements 62 at distal tip 28. The electrodes are generally configured for delivering ablation energy to tissue, and/or for sensing cardiac electrical signals. Catheter 14 additionally includes one or more position sensors 70 embedded in or near distal tip 28 for tracking position and orientation of distal tip 28. Position sensor 70 can be, for example, a magnetic-based position sensor, for example a position sensor including three magnetic coils for sensing three-dimensional (3D) position and orientation, or a position sensor including one magnetic coil, for sensing a single direction. One commercially available catheter that incorporates the features of the disclosed catheter is VARIPULSE^{™} pulsed field ablation (PFA) catheter available from Biosense Webster Inc., 31A Technology Drive, Irvine, CA 92618.

According to one example, electrical activity at points within the heart is typically sensed and measured by advancing a catheter containing one or more electrical sensors into a chamber of the heart, and acquiring data at a multiplicity of points. These data are then utilized to compute an anatomical map (or "electro-anatomical map") of the heart chamber or a portion thereof. According to another example, anatomical maps are imported into the system, such as those generated from an MRI or CT scan.

Magnetic-based position sensor 70 may be operated together with a location pad 25 including a plurality of magnetic coils 32 configured to generate magnetic fields in a predefined working volume. The real-time position of the distal end of a shaft of catheter 14 may be tracked based on magnetic fields generated with location pad 25 and sensed by magnetic-based position sensor 70. Details of the magnetic-based position sensing technology are described in U.S. Patent Nos. 5,539,199; 5,443,489; 5,558,091; 6,172,499; 6,239,724; 6,332,089; 6,484,118; 6,618,612; 6,690,963; 6,788,967; 6,892,091.

System 10 may further include one or more electrode patches 38 positioned for skin contact on patient 23 to establish location reference for location pad 25, as well as impedance-based tracking of electrodes 66. For impedance-based tracking, electrical current is directed to electrodes 66 and sensed at electrode skin patches 38 so that the location of each electrode can be triangulated via the electrode patches 38. Details of impedance-based location tracking technology are described in US Patent Nos. 7,536,218; 7,756,576; 7,848,787; 7,869,865; and 8,456,182.

Electrodes 66 can also be configured for receiving an AC signal while being paired with a reference electrode. Impedance in response to the AC signal may be sensed and used to determine local proximity between an electrode 66 and the tissue wall, e.g., cavity wall. An example method for assessing proximity based on impedance is described for example in US Patent Application No. 20210177504.

A recorder 11 can be utilized to record and display electrograms 21 captured with body surface ECG electrodes 18 and intracardiac electrograms (IEGMs) captured with electrodes 66 of catheter 14. Recorder 11 may include pacing capability for pacing the heart rhythm and/or may be electrically connected to a standalone pacer.

In some examples, system 10 includes an ablation energy generator 50 that is adapted to conduct ablative energy to electrodes 66 at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

In some examples, system 10 further includes patient interface unit (PIU) 30, which is an interface device configured to establish electrical communication between catheters, other electrophysiological equipment, power supply, and a workstation 55 for controlling operation of system 10. Electrophysiological equipment of system 10 may include, for example, multiple catheters, location pad 25, body surface ECG electrodes 18, electrode patches 38, ablation energy generator 50, and recorder 11. According to some examples, PIU 30 additionally includes processing capability for implementing real-time computations of the location of the catheters and for performing ECG calculations.

Workstation 55 includes a processing circuitry comprising one or more processors operatively connected to a computer memory of some sort. An appropriate operating software and user interface capability can be executed by the processing circuitry. Workstation 55 may provide multiple functions, optionally including, for example:
Modeling the endocardial anatomy in 3D and rendering a 3D graphical representation of the model ("3D anatomical map") 20 for display on a display device 27; displaying on display device 27 activation sequences, or other data, compiled from recorded electrograms 21 in representative visual indicia (e.g., by color coding) or imagery superimposed or overlaid on the rendered anatomical map 20; displaying real-time location and orientation of one or more catheters within the heart chamber; and displaying on display device 27 sites of interest such as places where ablation energy has been applied. One commercial product embodying elements of the system 10 is available as the CARTO^{™} 3 System, available from Biosense Webster.

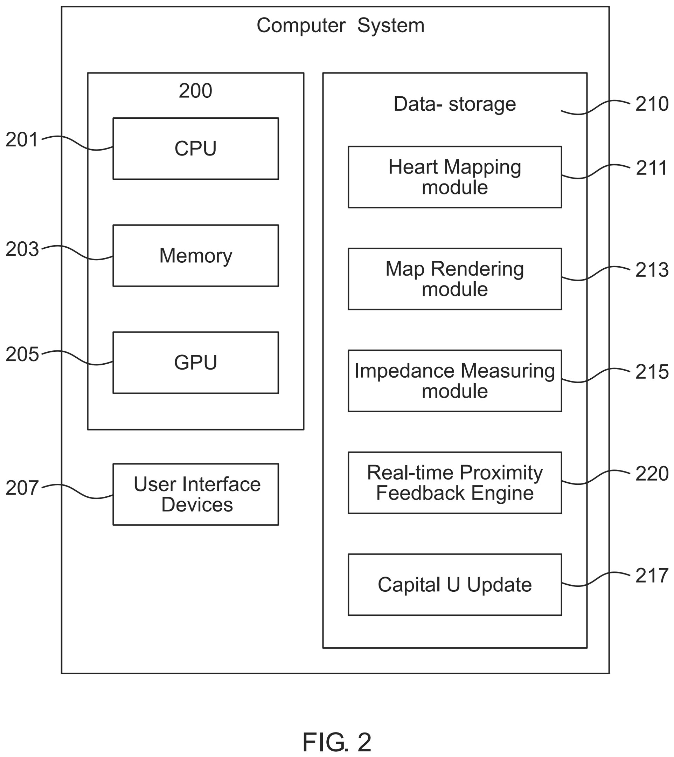

FIG. 2 illustrates, by way of example, a computer system configured to process, render, and display visual feedback of a catheter's proximity to tissue walls in real-time during Intraluminal Catheter Therapy (ICT). Such a computer system may, for instance, correspond to computer 55, shown as part of system 10 in FIG. 1. As further described below, the computer system is designed to provide dynamic and continuous proximity feedback by integrating measurements from the catheter's electrodes and projecting them onto a 3D anatomical map of the anatomical structure being examined. This enables clear visualization of the catheter's spatial relationship to the tissue walls.

System 55 show by way of example, processing circuitry 200, which includes at least one processing unit, such as a central processing unit (CPU 201) and/or a graphics processing unit (GPU 205), operatively connected to computer memory 203. According to the examples disclosed herein, the processing circuitry is configured to generate, render, and display a 3D anatomical map. This graphical representation serves as a visual reference for the anatomical structure (e.g., the heart) and supports real-time interaction with the catheter's positioning and proximity feedback.

The processing circuitry performs the calculations and other operations necessary for generating the 3D anatomical map, managing and displaying proximity data, and supporting system tasks. For instance, the CPU 201 may handle data processing and system coordination, whereas the GPU 205 may accelerate graphical rendering tasks, such as creating the anatomical map, applying textures, and overlaying real-time proximity feedback onto the 3D graphical representation.

Memory 203 is configured to store data required for system operation, including, for example, computer instructions dedicated for generating, updating, and displaying 3D graphical representations and proximity feedback. Additionally, data storage device 210 stores various types of data, such as computer programs, system files, and user interface software, which can be loaded into memory 203 during execution.

FIG. 2 illustrates, by way of example, several functional modules executable by computer 55, which are stored within data storage device 210 and loaded into memory 203 during operation to enable real-time processing and visualization. These functional modules include:
Heart Mapping Module 211, configured to map the internal luminal surface of the heart and generate a 3D model of the endocardial anatomy of the heart;
Map Rendering Module 213, configured to render a 3D graphical representation of the model and display it on a display device;
   Impedance Measuring Module 215, configured to receive impedance measurements from the electrodes; and

Real-time Proximity Feedback Engine 220, configured to calculate proximity values based on the measured impedance, and generate continuous visual (graphical) proximity feedback projected onto the 3D graphical representation of the heart. This integrated display reduces visual clutter, enabling physicians to efficiently assess and adjust the catheter's position relative to heart tissue in real-time.

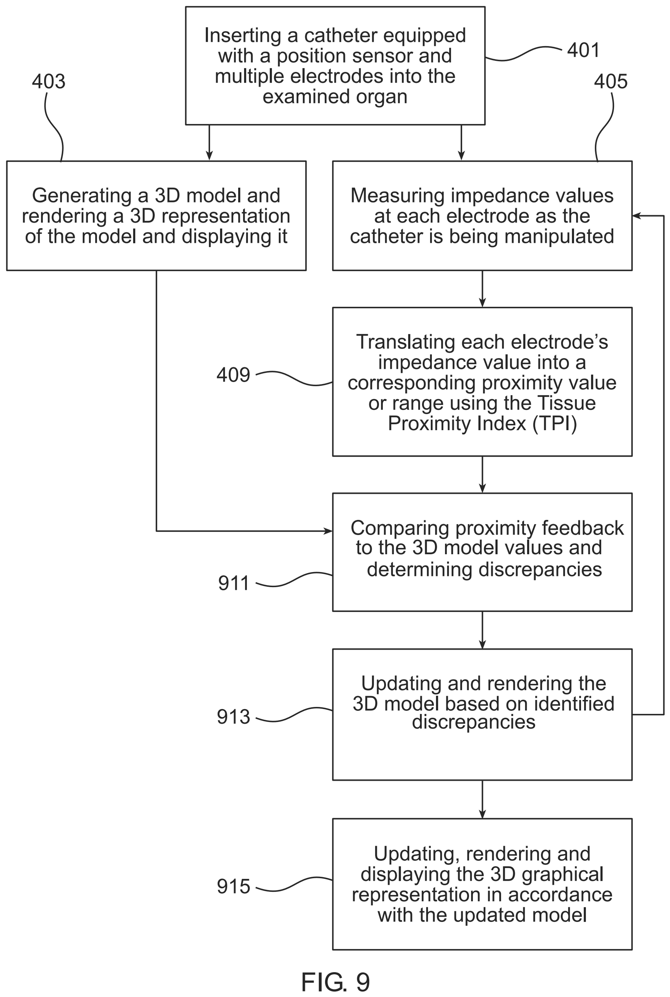

Optionally, computer system 55 includes a Mapping Update Module 217, configured to execute the proximity-derived dynamic model update procedure, as further described with reference to FIG. 9.

User Interface Devices 207, such as a display device (corresponding for example to display 27 shown in FIG. 1) and input tools like a mouse, keyboard, or touchscreen, are operatively connected to computer 55 to enable real-time visualization and interaction with the graphical display. This interface allows the physician to view the 3D graphical representation of the heart and simultaneously assess proximity feedback, facilitating real-time adjustments to catheter positioning.

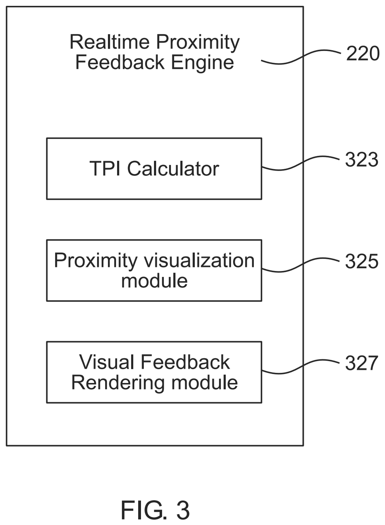

FIG. 3 illustrates a non-limiting example of the logical design of the Real-time Proximity Feedback Engine 220. The engine includes:
TPI Calculator 323, configured to calculate Tissue Proximity Index (TPI) values based on impedance measurements obtained from catheter electrodes; Proximity Visualization Module 325, configured to translate proximity values into corresponding graphical attributes; and Visual Feedback Rendering Module 327, configured to generate and render the continuous visual proximity feedback as disclosed herein.

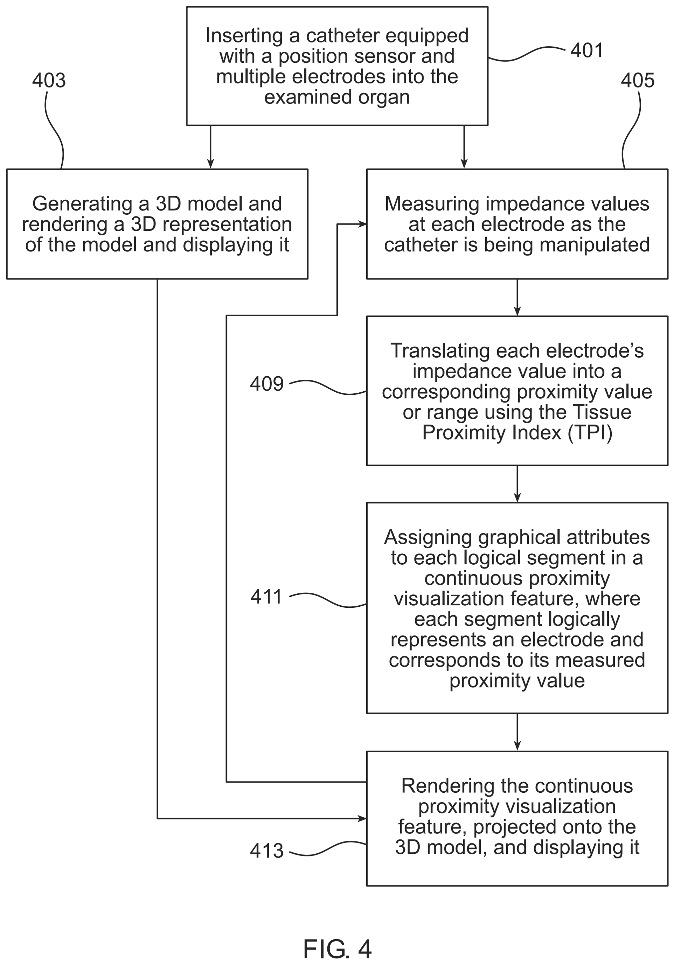

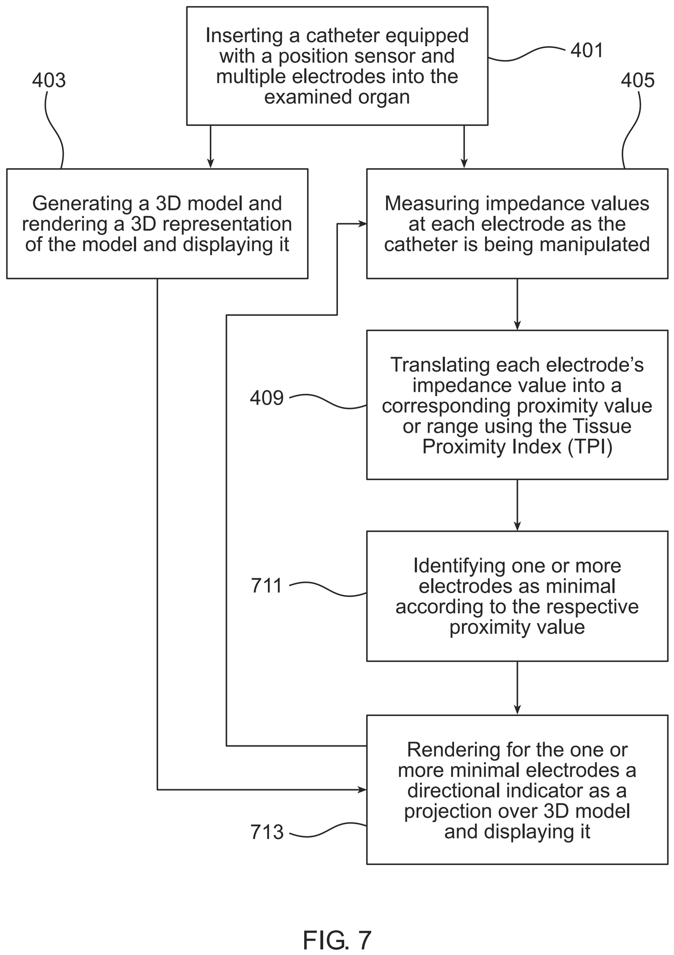

FIG. 4 is a flowchart illustrating the operations carried out for providing continuous visual proximity feedback, according to certain examples of the presently disclosed subject matter. For clarity and by way of non-limiting example, the description of operations in FIG. 4 (as well as FIGS. 7 and 9) is made with reference to components described in the previous figures.

During Intraluminal Catheter Therapy (ICT), a catheter equipped with a position sensor and electrodes is inserted into a specific compartment of the heart (block 401). The catheter maps the internal luminal surface of the heart to create a 3D model of the endocardial anatomy. As the medical practitioner navigates the catheter through various regions, the position sensor tracks its movements and transmits the position data, combined with input from external pads, to a computer. The recorded positions outline the contours of the internal structures of the heart. Based on this data, a 3D model of the heart is constructed (block 403; e.g., by the Heart Mapping Engine 211). The mapped data is then processed to render and display a 3D graphical representation ("3D anatomical map") of the model (e.g., by Map Rendering module 213).

Concurrently with the mapping of the heart, the catheter, equipped with multiple electrodes, measures impedance in real-time (block 405). Impedance serves as an indicator of each electrode's proximity to the tissue walls, with variations reflecting differences in the electrical properties of the tissue compared to other mediums, such as air or blood, that the electrode may contact when not in direct contact with the tissue. This real-time impedance measurement provides critical feedback on the level of contact between the catheter's distal assembly and the tissue wall of a luminal organ during procedures like Intraluminal Catheter Therapy (ICT).

As illustrated in FIG. 1, the catheter's distal assembly features a plurality of electrodes distributed along its length, forming a circumferential (or in other examples, an elongated) electrode array. Each electrode independently measures impedance, transmitting the data to the processing system for analysis (e.g., by Impedance Measuring module 215). As shown in FIG. 1 demonstrating a VARIPULSE^{™} catheter, in some configurations the electrodes are positioned on a contoured distal assembly, such as a circular or variable-loop structure. This configuration allows for the establishment of tissue contact of the electrodes over an approximately planar local contact region . This design facilitates precise contact mapping, while reducing data interpretation complexity.

The system processes the impedance data to generate real-time visual feedback that indicates the proximity of the electrodes to the tissue (e.g., by Real-Time Proximity Feedback Engine 220). As further explained below, a novel visual feedback feature, characterized by a continuous graphical element, is disclosed herein. This visual feedback allows the medical practitioner to assess the catheter's interaction with the tissue and make adjustments to its positioning or applied force. A low impedance value observed at a certain electrode or group of sequential electrodes may suggest insufficient contact with the tissue, prompting repositioning at that electrode location, while a high impedance value could indicate excessive pressure, requiring a reduction in force to prevent tissue damage. This system enhances the accuracy, safety, and effectiveness of the procedure by providing real-time insights into the catheter-tissue interaction.

Impedance values measured at each electrode are translated into their respective proximity values, indicating distance from the tissue wall, using a Tissue Proximity Index (TPI), which represents the relationship between impedance and proximity to the tissue wall (block 409; e.g., by TPI Calculator 323 in Engine 220).

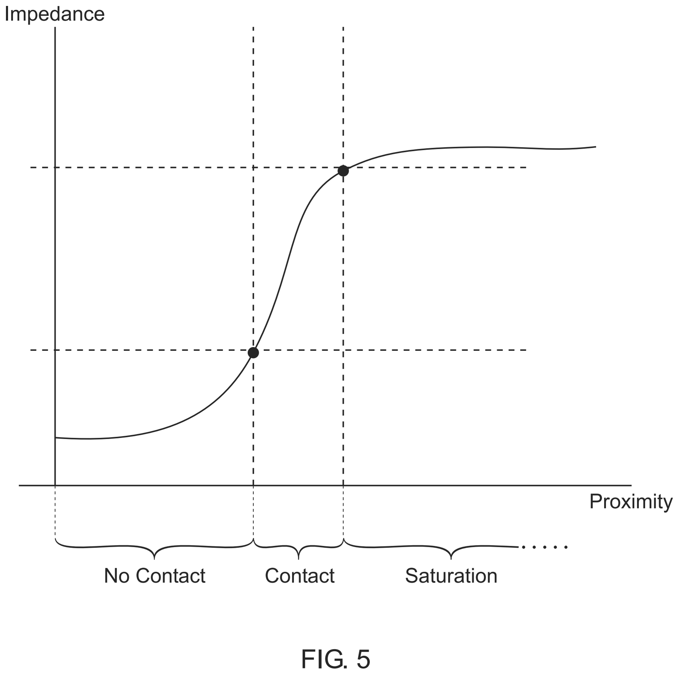

FIG. 5 is a graph depicting a TPI profile (impedance-proximity profile), where the x-axis corresponds to the proximity (inverse of distance) of an electrode to the tissue, and the y-axis corresponds to the impedance values measured by the electrode. Each electrode is characterized by a respective impedance profile, which reflects its interaction with the tissue.

As shown in FIG. 5, the graph is non-linear and can be divided into three distinct phases, including a non-contact phase, a contact phase, and a contact saturation phase. In the initial non-contact phase, the electrode is in contact with the blood and does not touch the tissue walls. At this phase, the measured impedance is relatively low. As the electrode is moved closer to the tissue wall the measured impedance increases. At a certain point marking the initial contact of the electrode with the tissue, a sharp increase in the measured impedance is observed. After this steep rise, the graph levels off into a plateau and enters a saturation phase. In this phase, the electrode is in full contact with the tissue, and the application of additional pressure does not result in a significant, if any, further increase in impedance measurements.

Each proximity value of each electrode, derived from the respective measured impedance, is translated to a corresponding graphical feature (block 411; e.g., by the Proximity Visualization Module 325 in Engine 220). These features are then visualized on the 3D anatomical map of the heart. This process ensures that proximity data is effectively and intuitively represented, enabling the physician to accurately assess and adjust catheter-tissue interaction in real-time.

For example, a graphical feature index can be used to define the relationship between proximity values or ranges of proximity values and graphical features and/or attributes used for their representation. This index can be defined according to various relationships. For instance, a linear relationship may be used, where graphical features (e.g., the diameter of a tube-like segment or the intensity of a color) change proportionally to the proximity values. Alternatively, a non-linear relationship may be employed to emphasize subtle changes in proximity at certain ranges.

In some examples, the graphical feature index may define a first threshold below which all proximity values are assigned a first graphical feature (e.g., blue shading indicating minimal contact). Similarly, proximity values exceeding a second threshold, higher than the first, may be assigned a second graphical feature (e.g., red shading indicating excessive pressure). For proximity values falling between the first and second thresholds, a plurality of distinct graphical features (e.g., varying shades of green) may be assigned, with the specific feature determined based on the exact proximity value.

According to the presently disclosed subject matter, the proximity data inferred from impedance measured by the electrodes is represented visually as a continuous graphical element (herein below "continuous proximity visualization feature") projected onto the 3D graphical representation of the model. Rather than indicating proximity by discrete graphical elements representing the electrodes themselves, the proximity feedback is represented as a continuous sequence of graphical elements, such as a ribbon, strip, or tube (e.g., with a rectangular or cylindrical cross-section), projected directly onto respective locations on the surface of the 3D anatomical map.

One example of a projection method involves sampling discrete points along the catheter's trajectory, corresponding to electrode positions (optionally including additional interpolated locations to improve trajectory resolution). These points are then projected onto the 3D anatomical surface by identifying the closest point on the surface for each sampled point, ensuring the catheter's trajectory is accurately aligned with the tissue geometry. To create a visually continuous representation, a mathematical smoothing curve, such as a quadratic B-spline, can be applied to the projected points, providing a smooth and continuous trajectory along the tissue surface. This projection approach is particularly applicable to circular catheters, such as the VARIPULSE^{™} catheter described above, where the sampled points correspond to circumferential electrode positions along the loop structure.

The rendering process (block 413; e.g., by the Visual Feedback Rendering module 327) integrates the continuous proximity visualization feature with the anatomical map in real-time. This visualization approach allows the physician to view the anatomical structure of the heart, together with seamless, real-time proximity feedback, in a clear and intuitive manner. By embedding proximity feedback directly onto the surface of the anatomical map, the system provides an integrated graphical representation that reduces visual clutter, enhances usability, and supports precise catheter positioning during procedures such as Intraluminal Catheter Therapy (ICT).

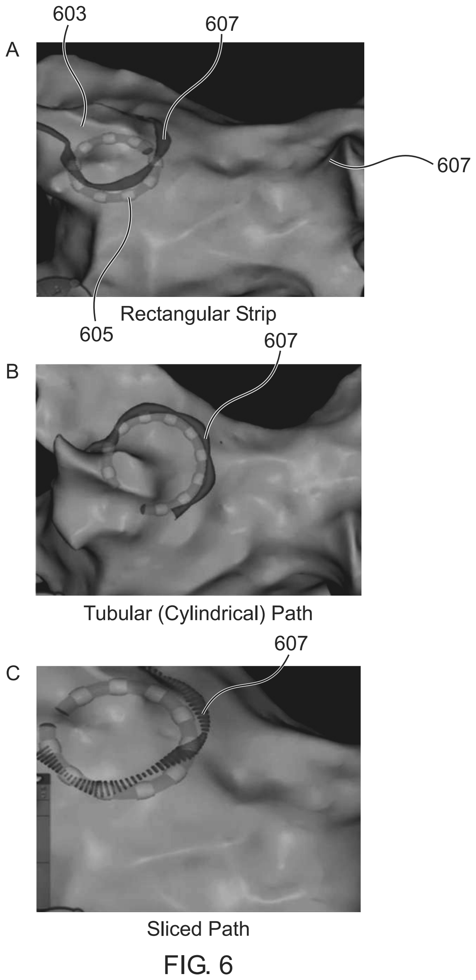

FIG. 6-A illustrates an example of a continuous proximity visualization feature within a heart chamber. A 3D graphical representation of the internal luminal space of the heart chamber is depicted (601), with an ablation catheter positioned inside a blood vessel (603). The graphical representation highlights the internal spatial structure of the heart chamber, providing clear visualization of the catheter's position relative to the luminal walls.

The catheter itself is represented by a graphical feature (605) consisting of a sequential pattern of segments, exhibiting for example, alternating colors. Segments of a first color correspond to the catheter's electrodes, while segments of another color represent the spaces between the electrodes.

A graphical representation of the catheter's proximity to the tissue is displayed, separately from the catheter's representation, as a continuous proximity visualization feature shaped as a strip with a rectangular cross-section, with the strip projected onto the 3D graphical representation of the tissue walls (607). Each segment or area along the strip is logically associated with a respective electrode. For example, different areas along the strip may correspond to sampled points linked to a specific electrode, creating a continuous graphical representation of proximity. The feature is rendered as a visually continuous feature to provide seamless proximity feedback. Graphical attributes such as width or color are used to indicate the proximity sensed by each electrode. For example, the graphical feature index may determine the width of the strip at each logical segment, based on the impedance measured by the corresponding electrode, e.g., according to associated sampled points. The relationship between the proximity values and the graphical feature values (e.g., width) can be defined by an appropriate graphical feature index, as explained with respect to block 411. Additionally, or alternatively, other graphical attributes, such as intensity or transparency, may be used to visualize proximity.

The dynamic variation of graphical attribute values displayed along the continuous strip provides a seamless visual indication of proximity differences, enabling the physician to assess and adjust the catheter's positioning in real-time. This method offers an intuitive and organized representation of catheter-tissue interaction, enhancing visual clarity and facilitating improved catheter adjustments. As a result, this approach improves the accuracy, safety, and efficacy of ICT procedures.
FIG. 6-B illustrates a second example of a continuous proximity visualization feature within a heart chamber. In this example, the feature (607) is characterized by a tubular cross-section projected onto the graphical representation of the tissue walls, providing a continuous representation of proximity along the catheter's trajectory.
FIG. 6-C illustrates a third example of a continuous proximity visualization feature within a heart chamber, where the feature is characterized by a sliced structure, such as a sliced strip or tube. In this example, proximity of the electrodes is indicated by varying the width of the sliced feature. Additionally, or alternatively, proximity can be represented by varying the spaces between the slices, where greater spacing indicates lower proximity value, and smaller spacing indicates higher proximity value. For example, each group of slices can correspond to a respective electrode, the spacing between adjacent slices in the group being determined and updated according to the proximity of the respective electrode. The same principles described above with respect to FIG. 6-A also apply to the examples of FIG. 6-B and 6-C.

The operations described with reference to blocks 405 to 413 are dynamically and continuously executed to reflect the varying proximity of the electrodes to the tissue walls in real-time, as the catheter is being manipulated within the heart. This ensures that the graphical attributes of the continuous proximity visualization feature are dynamically updated to reflect the varying proximity of the electrodes to the tissue walls as the catheter is being moved. Consequently, this provides continuous, real-time visual feedback to the medical practitioner. During a medical procedure, such as organ mapping or ablation, a physician can use the continuous proximity visualization displayed on-screen to guide the catheter, adjust tissue contact, and manage applied force in real-time, improving the procedure's accuracy and efficiency.

According to another example, instead of, or in addition to a continuous proximity visualization feature, the graphical visualization includes at least one directional indicator (e.g., arrow) to indicate electrodes on the catheter that are farther away from the tissue. This arrow is dynamically projected onto the anatomical map at positions corresponding to the electrodes with low proximity values. The arrow points towards the nearest location on the tissue surface, visually guiding the physician on how to adjust the catheter's position to improve contact with the tissue, thereby enhancing the precision and effectiveness of the procedure.

FIG. 7 is another flowchart illustrating the operations carried out for providing directional proximity feedback, according to certain examples of the presently disclosed subject matter.

The operations described in blocks 401, 403, 405, and 409 are similar to those discussed in detail with reference to FIG. 4. These include inserting the catheter equipped with a position sensor and multiple electrodes into the examined organ (block 401), generating and rendering a 3D anatomical map of the heart (block 403), measuring impedance values at each electrode (block 405), and translating these impedance values into corresponding proximity values using the Tissue Proximity Index (TPI) (block 709). For brevity, the reader is referred to the description of FIG. 4 for these operations.

Following these steps, FIG. 7 introduces new operations, beginning with the identification of one or more electrodes with minimal proximity values based on the translated proximity data (block 711; e.g., by TPI Calculator). This process involves analyzing the proximity data derived from the impedance measurements of each electrode using the Tissue Proximity Index (TPI). Proximity values that fall below a predefined threshold are flagged as minimal, indicating electrodes that are farthest from the tissue surface. By comparing the proximity values of all electrodes, the system identifies those electrodes with the lowest proximity values as those requiring improved contact with the tissue.

For the identified electrodes (possibly only one with the lowest proximity value), directional indicators are rendered and projected onto the 3D anatomical map (block 713; e.g., by Visual Feedback Rendering module 327). In some examples, a directional indicator is designed to point to the electrode with the largest distance from the surface, guiding the user to the weakest point or the area that most needs repositioning to achieve optimal proximity. These indicators, such as arrows or vectors, are displayed dynamically at positions corresponding to the identified electrodes. The arrows point toward the nearest point on the surface of the 3D anatomical map, providing the physician with visual guidance for adjusting the catheter's positioning to improve tissue contact. These operations enhance the visualization by adding actionable directional guidance, complementing the continuous proximity visualization feature described in FIG. 4. In such cases, operations described with respect to blocks 711 and 713 can be integrated within the process described with reference to FIG. 4. Notably, in some cases the directional guidance can be provided without the continuous proximity visualization.

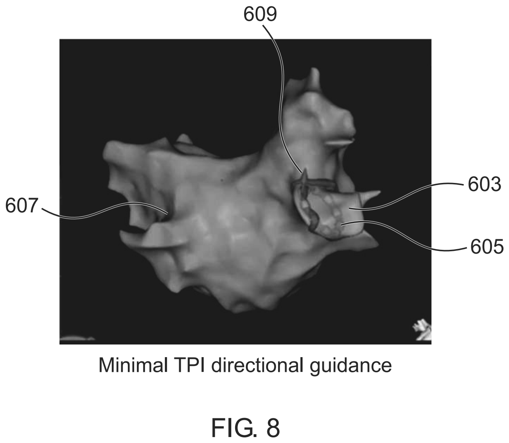

FIG. 8 illustrates an example of combining a continuous proximity visualization feature with directional proximity feedback indicators within a heart chamber. A 3D anatomical map of the internal luminal space of the heart chamber is depicted (601), with an ablation catheter (605) positioned inside a blood vessel (603). In addition to the continuous proximity visualization feature (607), projected onto the graphical representation of the tissue walls, a directional arrow (609) is projected onto the anatomical map, originating from an electrode identified with minimal proximity value and pointing toward the nearest tissue surface. By combining the continuous proximity visualization feature with these directional arrows, the system offers a comprehensive feedback mechanism. This integration allows the physician to assess overall catheter-tissue interaction, while receiving specific guidance on how to adjust the catheter for improved contact at critical areas.

In addition to the above-described techniques for real-time ICT proximity feedback, the presently disclosed subject matter further contemplates a computer-implemented method and a computer system for augmenting a 3D anatomical model of an examined organ during intraluminal catheter therapy (ICT).

According to the presently disclosed subject matter, it is suggested to harness the real-time ICT proximity feedback for augmenting the 3D anatomical model of the heart.

FIG. 9 is a flowchart illustrating operations carried out as part of the proximity-derived dynamic model update procedure, according to some examples of the presently disclosed subject matter.

The operations described in blocks 401, 403, 405, and 409 are similar to those discussed in detail with reference to FIG. 4. These include inserting the catheter equipped with a position sensor and multiple electrodes into the examined organ (block 401), generating and rendering a 3D graphical representation of the model (block 403), measuring impedance values at each electrode (block 405), and translating these impedance values into corresponding proximity values using the Tissue Proximity Index (TPI) (block 409). For brevity, the reader is referred to the description of FIG. 4 for these operations.

The 3D anatomical model generated by the position sensors may exhibit inaccuracies due to limitations in spatial resolution, calibration errors, or challenges in detecting complex anatomical structures. Consequently, the 3D anatomical map, which is based on the model, may inaccurately depict the catheter's position relative to the tissue walls, creating a false indication of its spatial location. This misrepresentation can distort the actual positioning of the catheter within the organ, leading to potential challenges in accurately guiding and monitoring the catheter during ICT, and potentially impacting accuracy and effectiveness.

The model-generation pathway based on the positioning system (blocks 401 and 403) operates in parallel with the proximity-estimation pathway (blocks 405 and 409), and the outputs of these pathways are subsequently compared.

The proximity feedback is compared to the 3D model to identify discrepancies that indicate mismatches between the modeled anatomy and the tissue contact inferred from the proximity data (911). Such discrepancies suggest incorrect mapping.

For example, if proximity feedback indicates full tissue contact, while the 3D model shows a gap between an electrode and the tissue wall, this suggests that the model does not accurately represent the actual anatomy.

Responsive to the identification of discrepancies, the computer system dynamically updates the 3D model by deforming or adjusting the mapped surfaces to interpolate the touching electrodes based on the proximity feedback (block 913). An updated map is then rendered and displayed in real time based on the updated model, ensuring an accurate representation of the organ's anatomy (block 915).

According to one example, the Mapping Update module 217 receives positive proximity values from TPI calculator 323 and adjusts the 3D model accordingly. These values represent the distance or contact level between the catheter's electrodes and the tissue wall. Using the TPI data, the Mapping Update module 217 deforms the mapped surfaces to align with the actual anatomical features, ensuring that the 3D model and its graphical representation more accurately reflect the true spatial structure of the mapped organ. For example, if it is determined, based on the proximity feedback, that an electrode is in full contact with the tissue wall, the area of the model corresponding to the contact point with this electrode can be made to collapse toward the electrode, reflecting the zero distance, and ensuring more accurate representation of the intraluminal structure of the organ.

Additionally, deformation of the mapped surface can be implemented as a continuous function of proximity values (e.g., by Mapping Update module 217). Areas with higher proximity values-indicating closer contact-can be deformed more significantly, interpolating toward the electrodes' positions. Conversely, areas with lower proximity values can be adjusted less, resulting in a smooth, continuous deformation across the mapped surface. This ensures a more accurate and realistic representation of the organ's anatomy, reflecting varying degrees of contact inferred from the proximity data. In some examples, a function defines how proximity values translate to surface deformation, ensuring smooth and proportional adjustments. For instance, a linear function may apply deformation directly proportional to proximity, while a non-linear function, such as exponential, can emphasize higher proximity values for sharper adjustments. These functions enable precise adaptation of the model to reflect anatomical structures accurately.

Following is a non-exclusive list of some exemplary examples of the disclosure. The present disclosure also includes examples which include fewer than all the features in an example and examples using features from multiple examples, even if not listed below.

Example 1: A computer-implemented method for providing real-time visual feedback of intraluminal catheter engagement using a catheter that comprises electrodes on a distal end assembly, the method comprising, while the catheter is positioned within a luminal organ of a patient:
generating a three-dimensional (3D) model of the internal surface of the luminal organ, based on positional data obtained by the catheter; rendering a 3D anatomical map graphically representing the 3D model;

For each electrode: determining a proximity value indicative of the distance between the electrode and a tissue wall of the luminal organ based at least on measured impedance; translating the proximity value into a corresponding graphical attribute value;
rendering a continuous proximity visualization feature that sequences the graphical attribute, with each graphical attribute corresponding to a location of a respective electrode, and where the corresponding graphical attribute values represent variations in proximity values across the electrodes;
projecting the continuous proximity visualization feature onto the surface of the luminal organ as depicted by the map, creating an integrated graphical representation;
displaying the integrated graphical representation on a computer display; and dynamically updating the visualization feature in response to changes in measured impedance, providing real-time feedback on catheter engagement with the tissue walls.

Example 2: The method according to Example 1, wherein determining a proximity value comprises applying a Tissue Proximity Index on the impedance values measured by the electrodes.

Example 3: The method according to any one of Examples 1 and 2, wherein translating the proximity value into a corresponding graphical attribute value comprises applying a graphical feature index that defines a relationship between proximity values or ranges of proximity values and graphical features and/or attributes values used for their representation.

Example 4: The method according to any one of Examples 1 to 3, wherein the continuous proximity visualization feature is rendered as a strip or tube-like shape; wherein each electrode corresponds to a respective area along the continuous proximity visualization feature that is rendered according to the graphical attributes assigned to the corresponding electrode.

Example 5: The method according to Examples 4, wherein the graphical attribute include one or more of: width, transparency, pattern, and color.

Example 6: The method according to Example 4, wherein the graphical attribute is the width of the continuous proximity visualization feature; wherein the width of each area within the feature is determined according to the proximity value of the respective electrode; and wherein variations in the proximity of different electrodes to the tissue wall induce variations in the width of the continuous proximity visualization feature, providing a visual representation of proximity differences.

Example 7: The method according to any one of Examples 1 to 6, wherein the continuous proximity visualization feature is rendered as a sliced shape, wherein each group of slices corresponds to a respective electrode, and the spacing between slices in a group is determined according to the proximity value of the respective electrode.

Example 8: The method according to any one of Examples 1 to 7 comprising:
identifying an electrode with a smallest proximity value based on the impedance values determined for each electrode;
rendering a directional guidance indicator on the 3D anatomical map, wherein the directional guidance indicator points from a location of the identified electrode towards an adjacent tissue wall, to thereby provide guidance for maneuvering the catheter to reduce the distance between the identified electrode and the tissue wall; and
dynamically updating the directional guidance indicator in real time based on changes in the proximity values, thereby enabling continuous guidance for optimizing catheter engagement with the tissue wall.

Example 9: The method according to any one of Examples 1 to 8 wherein the luminal organ is a heart of the patient.

Example 10: A computer system comprising a processing circuitry configured to execute operations according to any one of Examples 1 to 9.

Example 11: A non-transitory computer readable storage medium tangibly embodying a program of instructions that, when executed by a computer, cause the computer to perform a method according to any one of Examples 1 to 9.

Example 12: An Intraluminal catheter therapy (ICT) system that includes a computer system configured and operable to execute operation in accordance with any one of Examples 1 to 9.

Example 13: A computer-implemented method for providing real-time visual proximity feedback for intraluminal catheter engagement using a catheter comprising electrodes on a distal end assembly, the method comprising, while the catheter is positioned within a luminal organ of a patient:
mapping the internal surface of the luminal organ and generating a three-dimensional (3D) model of the internal surface;
rendering a graphical map of the 3D model as a visual representation of the luminal organ;
for each electrode determining a proximity value indicative of the distance between the electrode and a tissue wall of the luminal organ based on measured impedance;
identifying an electrode with the smallest proximity value;
rendering a directional guidance indicator on the 3D anatomical map, wherein the directional guidance indicator points from a location of the identified electrode towards an adjacent tissue wall, to thereby provide guidance for maneuvering the catheter to reduce the distance between the identified electrode and the tissue wall; and
dynamically updating the directional guidance indicator in real time based on changes in the proximity values, enabling continuous guidance for optimizing catheter engagement with the tissue wall.

Example 14: A computer-implemented method for augmenting a three-dimensional (3D) anatomical model of a luminal organ during intraluminal catheter therapy (ICT), the method comprising, while the catheter is positioned within a luminal organ of a patient:
generating a 3D anatomical model of the luminal organ based on positional data obtained from a position sensor;
rendering a graphical map of the 3D model as a visual representation of the luminal organ;
measuring impedance values at each electrode of the catheter;
determining, for each electrode, a proximity value indicative of the distance between the electrode and a tissue wall of the luminal organ based on the measured impedance;
comparing the proximity values to the 3D anatomical model to identify discrepancies between the catheter location as represented relative to the anatomical model and the catheter location inferred from the proximity feedback;
updating the 3D anatomical model by deforming or adjusting the mapped surfaces based on the proximity feedback to interpolate touching electrodes with the tissue surface, thereby augmenting the model to better represent actual anatomical features of the luminal organ; and
rendering and displaying the updated 3D anatomical model and respective graphical map in real time, providing an augmented representation of the luminal organ.

Example 15: A computer system comprising a processing circuitry configured to execute operations in accordance with any one of Examples 13 and 14.

Example 16: A non-transitory computer readable storage medium tangibly embodying a program of instructions that, when executed by a computer, cause the computer to perform a method in accordance with any one of Examples 13 and 14.

Example 17: An Intraluminal catheter therapy (ICT) system that includes a computer system configured and operable to execute operation in accordance with any one of Examples 13 and 14.

Example 18: A graphical user interface (GUI) for providing real-time visual feedback of intraluminal catheter engagement using a catheter comprising one or more electrodes positioned on a catheter distal end assembly. The GUI is executable by a computer to render and display a continuous proximity visualization feature in accordance with any one of Examples 1 to 9 and 13.

The term "luminal organ" refers to any organ that has a lumen, i.e., an interior space, cavity, or channel. Examples of luminal organs include blood vessels, kidneys, bladder, urethra, heart, and colon. It is noted that while the presently disclosed subject matter predominantly refers to Intraluminal Catheter Therapy (ICT) in the heart, this is provided by way of non-limiting example. The use of the disclosed innovations in ICT applications involving other luminal organs is also contemplated and falls within the scope of the disclosure.

Those skilled in the art to which the present disclosure pertains, can appreciate that while the present disclosure has been described in terms of preferred examples, the concept upon which this disclosure is based may readily be utilized as a basis for the designing of other structures, systems, and processes for carrying out the several purposes of the present disclosure.

The various illustrative logical blocks, modules, and algorithm steps, described in connection with the examples disclosed herein, can be implemented as electronic hardware, computer software, or combinations of both. To clearly illustrate this interchangeability of hardware and software, various illustrative components, blocks, modules, and steps are described generally in terms of their functionality. Whether such functionality is implemented as hardware or software depends upon the particular application and design constraints imposed on the overall system. The described functionality can be implemented in varying ways for each particular application, but such implementation decisions should not be interpreted as causing any departure from the disclosed subject matter.

Unless specifically stated otherwise, as apparent from the following discussions, it is appreciated that, throughout the specification, discussions utilizing terms such as "generating", "rendering", "measuring", "translating", "comparing", updating" or the like, include an action and/or processes of a computer that manipulate and/or transform data into other data, said data represented as physical quantities, e.g. such as electronic quantities, and/or said data representing the physical objects.

The terms "computer", "computer system", "computer device", or the like, should be expansively construed to include any kind of hardware-based electronic device with one or more data processing circuitries. Each processing circuitry can comprise, for example, one or more processors operatively connected to computer memory, (including non-transitory), loaded with executable instructions for executing operations, as further described herein.

The one or more processors referred to herein can represent, for example, one or more general-purpose processing devices, such as a microprocessor, a central processing unit, or the like. More particularly, a given processor may be one of: a complex instruction set computing (CISC) microprocessor, a reduced instruction set computing (RISC) microprocessor, a very long instruction word (VLIW) microprocessor, a processor implementing other instruction sets, or a processor implementing a combination of instruction sets. The one or more processors may also be one or more special-purpose processing devices such as an application-specific integrated circuit (ASIC), a field programmable gate array (FPGA), a digital signal processor (DSP), a graphics processing unit (GPU), a network processor, or the like.

FIG. 1, 2, and 3 illustrate schematics of the system architecture in accordance with certain examples of the presently disclosed subject matter. Elements in FIG. 1, 2, and 3 can be made up of any combination of software and hardware and/or firmware that performs the functions as defined and explained herein. Elements in FIG. 1, 2, and 3 may be centralized in one location, or dispersed over more than one location. In some examples, certain operations can be implemented by a remote cloud computing infrastructure, for example, where information is transmitted from computer 55 to the cloud where processing is executed, and the processing output is sent back to computer 55.

Also, it is to be understood that the phraseology and terminology employed herein are for the purpose of description, and should not be regarded as limiting. It should be noted that the words "comprising", "including" and "having" as used throughout the appended claims are to be interpreted to mean "including but not limited to". The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases, and disjunctively present in other cases. The term "each" may not be exclusively understood as referring to each and every, and when technically relevant may also refer to "at least some".

All patents and patent applications mentioned in this specification are herein incorporated in their entirety by reference into the specification, to the same extent, as if each individual patent or patent application were specifically and individually indicated to be incorporated herein by reference. In addition, citation or identification of any reference in this application shall not be construed as an admission that such reference is available as prior art to the present disclosure.

It will also be understood that the system according to the presently disclosed subject matter may be a suitably programmed computer. Likewise, the presently disclosed subject matter contemplates a computer program being readable by a computer for executing the method of the presently disclosed subject matter. The presently disclosed subject matter further contemplates a machine-readable (e.g., non-transitory) memory tangibly embodying a program of instructions executable by the machine for executing the method of the presently disclosed subject matter.

It is important, therefore, that the scope of the present disclosure is not construed as being limited by the illustrative examples set forth herein. Other variations are possible within the scope of the present disclosure as defined in the appended claims. Other combinations and sub-combinations of features, functions, elements, and/or properties may be claimed through amendment of the present claims or presentation of new claims in this or a related application. Such amended or new claims, whether they are directed to different combinations, or directed to the same combinations, whether different, broader, narrower, or equal in scope to the original claims, are also regarded as included within the subject matter of the present description.

## Claims

1. A computer-implemented method for providing real-time visual feedback of intraluminal catheter engagement, while using a catheter comprising electrodes on a distal end assembly, the method comprising, while the catheter is positioned within a luminal organ of a patient:
generating a three-dimensional (3D) model of an internal surface of the luminal organ;
rendering a 3D anatomical map graphically representing the 3D model;
for each electrode:
determining a proximity value indicative of the distance between the electrode and a tissue wall of the luminal organ based at least on measured impedance;
translating the proximity value into a corresponding graphical attribute value;
rendering a continuous proximity visualization feature that sequences the graphical attribute, with each graphical attribute corresponding to a location of a respective electrode, and where the corresponding graphical attribute values represent variations in proximity values across the electrodes;
projecting the continuous proximity visualization feature onto the surface of the luminal organ as depicted by the map, creating an integrated graphical representation;
displaying the integrated graphical representation on a computer display; and
dynamically updating the visualization feature in response to changes in measured impedance, providing real-time feedback on catheter engagement with the tissue wall.

2. The method of claim 1, wherein determining a proximity value comprises applying a Tissue Proximity Index on the impedance values measured by the electrodes.

3. The method of any one of the preceding claims, wherein translating the proximity value into a corresponding graphical attribute value comprises applying a graphical feature index that defines a relationship between proximity values or ranges of proximity values, and graphical features and/or attributes values used for their representation.

4. The method of any one of the preceding claims, wherein:
the continuous proximity visualization feature is rendered as a strip or tube-like shape; wherein each electrode corresponds to a respective area along the continuous proximity visualization feature that is rendered according to the graphical attributes assigned to the corresponding electrode.

5. The method of claim 4, wherein the graphical attribute includes one or more of: width, transparency, pattern, and color.

6. The method of claim 4, wherein the graphical attribute is the width of the continuous proximity visualization feature; wherein the width of each area within the feature is determined according to the proximity value of the respective electrode; and wherein variations in the proximity of different electrodes to the tissue wall induce variations in the width of the continuous proximity visualization feature, providing a visual representation of proximity differences.

7. The method of any one of the preceding claims, wherein the continuous proximity visualization feature is rendered as a sliced shape, wherein each group of slices corresponds to a respective electrode, and the spacing between slices in a group is determined according to the proximity value of the respective electrode.

8. The method of any one of the preceding claims further comprising:
identifying an electrode with a smallest proximity value based on the impedance values determined for each electrode;
rendering a directional guidance indicator on the 3D anatomical map, wherein the directional guidance indicator points from a location of the identified electrode towards an adjacent tissue wall, to thereby provide guidance for maneuvering the catheter to reduce the distance between the identified electrode and the tissue wall; and
dynamically updating the directional guidance indicator in real-time based on changes in the proximity values, thereby enabling continuous guidance for optimizing catheter engagement with the tissue wall.

9. The method of any one of the preceding claims wherein the luminal organ is a heart of the patient.

10. A computer system comprising a processing circuitry configured to execute operations according to any one of claims 1 to 9.

11. A non-transitory computer-readable storage medium tangibly embodying a program of instructions that, when executed by a computer, cause the computer to perform a method according to any one of claims 1 to 9.

12. A computer-implemented method for providing real-time visual proximity feedback for intraluminal catheter engagement using a catheter comprising electrodes on a distal end assembly, the method comprising, while the catheter is positioned within a luminal organ of a patient:
generating a three-dimensional (3D) model of the internal surface based on positional data obtained by the catheter;
rendering a graphical map of the 3D model as a visual representation of the luminal organ;
for each electrode, determining a proximity value indicative of the distance between the electrode and a tissue wall of the luminal organ based on measured impedance;
identifying an electrode with the smallest proximity value;
rendering a directional guidance indicator on the 3D anatomical map, wherein the directional guidance indicator points from a location of the identified electrode towards an adjacent tissue wall, to thereby provide guidance for maneuvering the catheter to reduce the distance between the identified electrode and the tissue wall; and
dynamically updating the directional guidance indicator in real-time based on changes in the proximity values, enabling continuous guidance for catheter engagement with the tissue wall.

13. A computer-implemented method for augmenting a three-dimensional (3D) anatomical model of a luminal organ during intraluminal catheter therapy (ICT), the method comprising, while the catheter is positioned within a luminal organ of a patient:
generating a three-dimensional (3D) model of the internal surface based on positional data obtained by the catheter;
rendering a graphical map of the 3D model as a visual representation of the luminal organ;
measuring impedance values at each electrode of the catheter;
determining, for each electrode, a proximity value indicative of the distance between the electrode and a tissue wall of the luminal organ, based on the measured impedance;
comparing the proximity values to the 3D anatomical model to identify discrepancies between the catheter location as represented relative to the anatomical model and the catheter location inferred from the proximity feedback;
updating the 3D anatomical model by deforming or adjusting the mapped surfaces, based on the proximity feedback, to interpolate touching electrodes with the tissue surface, thereby augmenting the model to better represent actual anatomical features of the luminal organ; and
rendering and displaying the updated 3D anatomical model and respective graphical map in real-time, providing an augmented representation of the luminal organ.

14. A computer system comprising a processing circuitry configured to execute operations according to any one of claims 12 and 13.

15. A non-transitory computer-readable storage medium tangibly embodying a program of instructions that, when executed by a computer, cause the computer to perform a method according to any one of claims 12 and 13.
